# EUROPEAN PATENT APPLICATION

(11) **EP 4 741 822 A2**
(43) Date of publication of application: **13.05.2026**
(21) Application number: 25214017.3
(22) Date of filing: 06.11.2025
(51) Int. Cl.: G01N 33/00, F01N 3/20

(54) **EMISSION APPARATUSES FOR SENSOR CROSS-SENSITIVITY TO AMMONIA AND ASSOCIATED SYSTEMS AND METHODS**

(30) Priority: 11.11.2024 US 202463718856 P
(71) Applicant: Safety Power Inc., Mississauga ON L4W 5A1 (CA)
(72) Inventor: Stelzer, Robert M, Mississauga, L4W 5A1 (CA); Puthuparampil, Jobin, Mississauga, L4W 5A1 (CA); Pong, Henry Ho Yin, Mississauga, L4W 5A1 (CA); Ostaltsov, Andrey, Mississauga, L4W 5A1 (CA)
(74) Representative: Slingsby Partners LLP

(57) **Abstract**

Provided are embodiments for a sensor apparatus for measuring emissions, including: an extraction member (104) in fluid communication with the exhaust gas flow (114), the extraction member (104) having an inlet end (122) and an outlet end (124), the inlet end (122) receiving an exhaust gas sample from the exhaust gas flow (114); an ammonia capture media (110) positioned downstream of the inlet end (122) within the extraction member (104), the ammonia capture media (110) comprising a sorbent configured to remove ammonia from the exhaust gas sample; and a post-capture sensor (106b) positioned downstream of the ammonia capture media (110), the post-capture sensor (106b) configured to measure at least one downstream NOx concentration measurement in the exhaust gas sample of the exhaust gas flow (114). Further provided are associated systems and methods for measuring emissions.

## Description

### FIELD

The present embodiments relate generally to emission sensing for combustion devices such as boilers, generators and internal combustion engines. More specifically, the present embodiments are directed to an emission apparatus for improving the operation of emissions sensors where such sensors are cross-sensitive to ammonia.

### BACKGROUND

Exhaust gas emission control has become particularly important due to stringent regulatory emission limits on boilers, generators and reciprocating engines. A typical exhaust gas after-treatment system may comprise many different individual emission reduction functions in order to meet the regulatory emission standards. More specifically, the selective catalytic reduction (SCR) device is frequently used in the exhaust system of combustion devices to eliminate particles of nitrogen oxides (NOₓ) in the exhaust gas. The SCR device is normally located in the exhaust system downstream of the combustion that takes place in a boiler, generator or reciprocating engine. The SCR device contains a SCR catalyst to reduce NOₓ, particles in the exhaust gas as the SCR catalyst must be heated before it can be used to reduce NOₓ, particles. In other words, until the SCR catalyst reaches an activation temperature, which is the minimum temperature to which the SCR catalyst must be heated, the SCR catalyst does not provide NOₓ, emission reduction. Although the hot exhaust gas from the combustion in a boiler, generator or reciprocating engine heats up the SCR Catalyst, for certain applications the length of time required to heat up the SCR Catalyst using hot exhaust gas alone can be too long.

SCR technology relies on a chemical reaction, which occurs between 260-540°C (500-1000F) to reduce NOₓ, particles in the exhaust gas. This commonly includes the use of gaseous ammonia provided by urea solution (i.e. CH₄N₂O or CO(NH₂)₂). Injecting gaseous ammonia is important because it is used as a reactant for reducing the NOₓ emissions at the SCR. The most common emission reduction reaction in the SCR of several NOₓ variants is 4NO + 4NH₃ + O₂ → 4N₂ + 6H₂O.

Nitrogen oxides (NOₓ) emission sensor devices are used to measure the NOx emission concentrations within the exhaust gas flow in order to provide measurement data for the operation of the emissions system. This can include measurements for controlling the injection of liquid urea or liquid ammonia solution to generate gaseous ammonia to permit the emission control reaction at the SCR device. These sensors however, are commonly cross-sensitive to gaseous ammonia and therefore face challenges with providing accurate measurement data. Cross-sensitivity refers to a phenomenon that occurs when a gas other than the gas being monitored causes the gas sensor to show a reading, even when the target gas is not present. This cross-sensitive property can interfere with the accuracy of the sensor and thus prevent effective control or fine tuning of an emission control system.

The injection of liquid urea or liquid ammonia solution into the exhaust stream also faces a challenge related to so-called "ammonia slip". Ammonia slip refers to the condition wherein unreacted ammonia (NH₃), introduced as a reductant into the selective catalytic reduction (SCR) system for the purpose of reducing nitrogen oxides (NOₓ), passes through the catalyst without participating in the intended chemical reactions and is subsequently emitted in the exhaust stream. Ammonia slip may result from factors such as reductant over-dosing, insufficient catalyst activity, non-uniform reductant distribution, or suboptimal temperature conditions within the catalyst zone.

Conventional NOₓ monitoring sensors address the ammonia/NOₓ cross-sensitivity problem by adjustment of the measurement of either the NOₓ or ammonia flow rate upstream of the SCR catalyst and monitoring the resulting downstream sensor reading, sometimes referred to as sensor dithering. The dithering approach is a poor solution since the NOₓ and/or ammonia concentrations in the exhaust flow would be unbalanced for a period of time during the dithering process, which may lead to issues meeting very stringent NOₓ emission targets.

There is a need therefore for improved emission apparatuses to improve sensor accuracy.

### SUMMARY

It is desirable to have a sensor apparatus for improving the precision of NOₓ measurements from an exhaust stream where gaseous ammonia is used to reduce the NOₓ emissions. This includes the fact that the improved precision of measurements would improve the control model for the emission reductions system. Downstream NOₓ sensor data provides a "feedback" into a control system that operates the emission reduction system, including corrections based on field conditions. The ammonia injection thus can interfere with post-capture sensor readings to cause an "inverse control" problem.

The present embodiments provide a solution to the ammonia/NOx cross-sensitivity problem differently by providing a sorbent material to remove the ammonia from the exhaust sample prior to measuring the NOₓ downstream in order to target much lower NOₓ levels at the outlet.

With the present embodiments, a feedback control loop receives a more accurate NOₓ reading from a post-capture sensor which is used to adjust the upstream urea injection rate ahead of the SCR device. This improve the accuracy of the NOₓ control system and allow for improved NOₓ emission reductions.

In a first aspect, there is provided a sensor apparatus for measuring emissions, the apparatus comprising: an extraction member in fluid communication with the exhaust gas flow, the extraction member having an inlet end and an outlet end, the inlet end receiving an exhaust gas sample from the exhaust gas flow; an ammonia capture media positioned downstream of the inlet end within the extraction member, the ammonia capture media comprising a sorbent configured to remove ammonia from the exhaust gas sample; and a post-capture sensor positioned downstream of the ammonia capture media, the post-capture sensor configured to measure at least one downstream NOx concentration measurement in the exhaust gas sample of the exhaust gas flow.

In one or more embodiments, the apparatus may further include an pre-capture sensor positioned between the inlet end and the ammonia capture media and configured to measure at least one upstream NOx concentration measurement in the exhaust gas sample.

In one or more embodiments, at least one of the pre-capture sensor and the post-capture sensor may be cross-sensitive to ammonia.

In one or more embodiments, the apparatus may further include a collector assembly connected to the inlet end, the collector assembly may include at least two sample inlets in communication with the exhaust gas flow, a first sample inlet in the at least two sample inlets receiving a first portion of the exhaust gas sample at a first location of the exhaust gas flow and a second sample inlet in the at least two sample inlets receiving a second portion of the exhaust gas sample at a second location of the exhaust gas flow.

In one or more embodiments, the apparatus may further include an eductor or a pump configured to increase a flow rate of the exhaust gas sample.

In one or more embodiments, the apparatus may further include a pump receiving the exhaust gas sample from the post-capture sensor, the pump configured to increase a flow rate of the exhaust gas sample.

In one or more embodiments, the at least one downstream NOx concentration measurement and the at least one upstream NOx concentration measurement may include at least one selected from the group of: nitric oxide and nitrogen dioxide.

In one or more embodiments, the at least one downstream NOx concentration measurement and the at least one upstream NOx concentration measurement may include at least one selected from the group of: nitric acid, nitrous acid, dinitrogen pentoxide, peroxyacetyl nitrate, alkyl nitrates, peroxyakyl bitrates, nitrate radical, and peroxynitric acid.

In one or more embodiments, the outlet end may be downstream of the post-capture sensor and is in fluid communication with the exhaust gas flow.

In one or more embodiments, the outlet end may be downstream of the post-capture sensor and comprises an atmospheric outlet.

In one or more embodiments, the ammonia capture media may further include: a sorbent housing in communication with the exhaust gas; wherein the sorbent may be removable from the sorbent housing.

In one or more embodiments, the ammonia capture media may include: a removable sorbent housing in communication with the exhaust gas; wherein the sorbent may be positioned within the removable sorbent housing .

In one or more embodiments, the selective sorbent may be phosphoric acid.

In one or more embodiments, the selective sorbent may be a condenser.

In one or more embodiments, the apparatus may further include a heater for heating the first and the post-capture sensor to an operating temperature.

In one or more embodiments, the pre-capture sensor and the post-capture sensor may receive a heating airflow from the inlet end of the extraction member.

In one or more embodiments, the heating airflow may be the exhaust gas sample.

In one or more embodiments, the ammonia capture housing may be heated to an operating temperature by an external heat source.

In one or more embodiments, the ammonia capture housing may receive a heating airflow from the inlet end of the extraction member.

In one or more embodiments, the heating airflow may be the exhaust gas sample.

In one or more embodiments, the inlet end of the extraction member may be positioned downstream of an SCR device.

In a second aspect, there is provided a system for reducing emissions in an exhaust gas flow, the system comprising: an ammonia capture media in an extraction member, an inlet end of the extraction member in fluid communication with the exhaust gas flow and receiving an exhaust gas sample from the exhaust gas flow, the ammonia capture media for reducing gaseous ammonia in the exhaust gas sample; a post-capture sensor positioned downstream of the ammonia capture media, the post-capture sensor configured to measure at least one downstream NOx concentration measurement of the exhaust gas sample; a processor in communication with the post-capture sensor and an ammonia generation means the processor configured to: receive the at least one downstream NOx concentration measurement from the post-capture sensor; determine an aggregate NOx concentration measurement based on the at least one downstream NOx concentration measurement; and transmit a control signal to an ammonia producing means based on the aggregate NOx concentration measurement, the ammonia producing means is positioned in the exhaust gas flow upstream of the extraction member.

In one or more embodiments, the system may further include an pre-capture sensor positioned between the inlet end and the ammonia capture media and configured to measure at least one upstream NOx concentration measurement in the exhaust gas sample; and wherein the processor may be further configured to receive the at least one upstream NOx concentration measurement and determine the aggregate NOx concentration measurement based on the at least one upstream NOx concentration measurement and the at least one downstream NOx concentration measurement.

In one or more embodiments, at least one of the pre-capture sensor and the post-capture sensor may be cross-sensitive to ammonia.

In one or more embodiments, the at least one downstream NOx concentration measurement and the at least one upstream NOx concentration measurement may include at least one selected from the group of: nitric oxide and nitrogen dioxide.

In one or more embodiments, the at least one downstream NOx concentration measurement and the at least one upstream NOx concentration measurement may further include at least one selected from the group of: nitric acid, nitrous acid, dinitrogen pentoxide, peroxyacetyl nitrate, alkyl nitrates, peroxyakyl bitrates, nitrate radical, and peroxynitric acid.

In one or more embodiments, the processor may be configured to store in a memory at least one selected from the group of: the aggregate NOx concentration measurement, the at least one upstream NOx concentration measurement and the at least one downstream NOx concentration measurement.

In one or more embodiments, the processor may be configured to determine an ammonia concentration based on at least one selected from the group of: the aggregate NOx concentration measurement, the at least one upstream NOx concentration measurement and the at least one downstream NOx concentration measurement.

In one or more embodiments, the processor may be configured to send a control signal to a pump receiving the exhaust gas sample.

In one or more embodiments, the processor may be configured to: determine a state of the ammonia capture media.

In one or more embodiments, the processor may be configured to communicate the state of the ammonia capture media.

In one or more embodiments, the processor may be configured to determine the control signal for the ammonia producing means based on the aggregate NOx concentration measurement and the state of the ammonia capture media.

In one or more embodiments, the system may further include: a pre-capture sensor external heat source and a post-capture sensor external heat source; wherein the processor may be configured to send a control signal to the pre-capture sensor external heat source and post-capture sensor external heat source.

In one or more embodiments, the inlet end of the extraction member may be positioned downstream of an SCR device.

In one or more embodiments, the system may further include: an isolation device upstream from the ammonia capture media controlling the first exhaust gas sample; a secondary sensor positioned in a second exhaust gas sample; and wherein the processor may be further configured to: receive a signal from the secondary sensor; and responsive to the signal from the secondary sensor, send a signal to the isolation device to permit the first exhaust gas sample to flow.

In one or more embodiments, the system may further include a secondary sensor positioned in a second exhaust gas sample; and wherein the processor may be further configured to: receive a signal from the secondary sensor; and responsive to the signal from the secondary sensor, send a signal to a pump or an eductor to permit the first exhaust gas sample to flow.

In a third aspect, there is provided a method for reducing emissions in an exhaust gas flow, the method comprising: extracting, from the exhaust gas flow into an inlet of an extraction member, an exhaust gas sample; reducing, using an ammonia capture media within the extraction member, gaseous ammonia from the exhaust gas sample; measuring, at a post-capture sensor positioned downstream from the ammonia capture media, at least one downstream NOx concentration measurement of the exhaust gas sample; receiving, at a processor, the at least one downstream NOx measurement; determining, at the processor, an aggregate NOx concentration measurement based on the at least one downstream NOx concentration measurement; and transmitting, from the processor to an ammonia producing means positioned in the exhaust gas flow, a control signal based on the aggregate NOx concentration measurement.

In one or more embodiments, the method may further include: measuring, at an pre-capture sensor positioned between the inlet of the extraction member and the ammonia capture media, at least one upstream NOx concentration measurement of the exhaust gas sample; receiving, at the processor, the at least one upstream NOx measurement; wherein the aggregate NOx concentration measurement is determined based on the at least one downstream NOx concentration measurement and the at least one upstream NOx concentration measurement.

In one or more embodiments, at least one of the pre-capture sensor and the post-capture sensor may be cross-sensitive to ammonia.

In one or more embodiments, the at least one downstream NOx concentration measurement and the at least one upstream NOx concentration measurement may include at least one selected from the group of: nitric oxide and nitrogen dioxide.

In one or more embodiments, the at least one downstream NOx concentration measurement and the at least one upstream NOx concentration measurement may further include at least one selected from the group of: nitric oxide, nitrogen dioxide, nitric acid, nitrous acid, dinitrogen pentoxide, peroxyacetyl nitrate, alkyl nitrates, peroxyakyl bitrates, nitrate radical, and peroxynitric acid.

In one or more embodiments, the method may further include storing in a memory at least one selected from the group of: the aggregate NOx concentration measurement, the at least one upstream NOx concentration measurement and the at least one downstream NOx concentration measurement.

In one or more embodiments, the method may further include determining an ammonia concentration based at least one selected from the group of: the aggregate NOx concentration measurement, the at least one upstream NOx concentration measurement and the at least one downstream NOx concentration measurement.

In one or more embodiments, the method may further include sending a control signal to a pump receiving the exhaust gas sample.

In one or more embodiments, the method may further include determining, at the processor, a state of the ammonia capture media.

In one or more embodiments, the method may further include transmitting, from the processor to a network device, the state of the ammonia capture media to a network device.

In one or more embodiments, the method may further include determining the control signal for the ammonia producing means based on the aggregate NOx concentration measurement and the state of the ammonia capture media.

In one or more embodiments, the method may further include: a first sensor external heat source and a post-capture sensor external heat source; wherein the processor may be configured to send a control signal to the first sensor external heat source and post-capture sensor external heat source.

In one or more embodiments the method may further include: receiving a signal from a secondary sensor positioned in a second exhaust gas sample; responsive to the signal from the secondary sensor, sending a signal to an isolation device upstream from the ammonia capture media, the signal to the isolation device permitting the first exhaust gas sample to flow.

### DRAWINGS

FIG. 1 shows a side view of an assembly with the extraction member that returns the exhaust gas sample to the exhaust gas flow including two sensors and ammonia capture media in accordance with one or more embodiments.
FIG. 2 shows a system view of the assembly from FIG. 1 with reference to the SCR and an combustion device in accordance with one or more embodiments.
FIG. 3 shows a system view of the assembly with reference to the SCR and an combustion device but with the extraction member venting the exhaust gas sample to the atmosphere in accordance with one or more embodiments.
FIG. 4 shows a side view of the assembly with a pump placed before the pre-capture sensor in accordance with one or more embodiments.
FIG. 5 shows a side view of the assembly with a pump placed after the post-capture sensor in accordance with one or more embodiments.
FIG. 6A shows an expanded view of the ammonia capture media showing removable sorbent housing containing the sorbent material in accordance with one or more embodiments.
FIG. 6B shows a cross-section view of an alternate embodiment of the ammonia capture media.
FIG. 7 shows a side view of a sample collection assembly with a plurality of sample inlets in accordance with one or more embodiments.
FIG. 8 shows a side view of the assembly with a heater supplying heat to the upstream and post-capture sensor and ammonia capture media in accordance with one or more embodiments.
FIG. 9 shows a side view of the assembly with a heating air flow heating the sensors and ammonia capture media in accordance with one or more embodiments.
FIG. 10 shows a side view of the assembly with a processor and a network device in communication with the sensors in accordance with one or more embodiments.
FIG. 11 shows the side view of FIG. 10 including an ammonia producing means connected to the processor in accordance with one or more embodiments.
FIG. 12 shows a method diagram in accordance with one or more embodiments.
FIG. 13 shows a device diagram in accordance with one or more embodiments.
FIG. 14 shows a results diagram showing the performance of the sensor assembly of the present embodiments including the ammonia capture media.
FIG. 15 shows a side view of another sample collection assembly in accordance with one or more embodiments.
FIG. 16 shows another side view of an assembly with the extraction member that returns the exhaust gas sample to the exhaust gas flow including two sensors and ammonia capture media, and additionally an independent extraction member in accordance with one or more embodiments.

### DESCRIPTION OF VARIOUS EMBODIMENTS

Various embodiments will now be described below to provide an example of the claimed subject matter. No example described below limits any claimed subject matter and any claimed subject matter may cover embodiments such as systems or methods that differ from those described below.

Furthermore, it will be appreciated that for simplicity and clarity of illustration, where considered appropriate, reference numerals may be repeated among the figures to indicate corresponding or analogous elements. In addition, numerous specific details are set forth in order to provide a thorough understanding of the examples described herein. However, it will be understood by those of ordinary skill in the art that the examples described herein may be practiced without these specific details. In other instances, well-known methods, procedures and components have not been described in detail so as not to obscure the examples described herein. Also, the description is not to be considered as limiting the scope of the examples described herein.

It should also be noted that, as used herein, the wording "and/or" is intended to represent an inclusive-or. That is, "X and/or Y" is intended to mean X or Y or both, for example. As a further example, "X, Y, and/or Z" is intended to mean X or Y or Z or any combination thereof.

It should be noted that terms of degree such as "substantially", "about" and "approximately" as used herein mean a reasonable amount of deviation of the modified term such that the end result is not significantly changed. These terms of degree may also be construed as including a deviation of the modified term if this deviation would not negate the meaning of the term it modifies.

Furthermore, the recitation of numerical ranges by endpoints herein includes all numbers and fractions subsumed within that range (e.g., 1 to 5 includes 1, 1.5, 2, 2.75, 3, 3.90, 4, and 5). It is also to be understood that all numbers and fractions thereof are presumed to be modified by the term "about" which means a variation of up to a certain amount of the number to which reference is being made if the end result is not significantly changed.

Some elements herein may be identified by a part number, which is composed of a base number followed by an alphabetical or subscript-numerical suffix (e.g., 112a, or 112₁). Multiple elements herein may be identified by part numbers that share a base number in common and that differ by their suffixes (e.g., 112₁, 112₂, and 112₃). All elements with a common base number may be referred to collectively or generically using the base number without a suffix (e.g., 112).

The example systems and methods described herein may be implemented in hardware or software, or a combination of both. In some cases, the examples described herein may be implemented, at least in part, by using one or more computer programs, executing on one or more programmable devices comprising at least one processing element, a data storage element (including volatile and non-volatile memory and/or storage elements), and at least one communication interface. These devices may also have at least one input device (e.g., a keyboard, a mouse, a touchscreen, and the like), and at least one output device (e.g., a display screen, a printer, a wireless radio, and the like) depending on the nature of the device. For example, and without limitation, the programmable devices (referred to below as computing devices) may be a server, network appliance, embedded device, computer expansion module, a personal computer, laptop, personal data assistant, cellular telephone, smart-phone device, tablet computer, a wireless device or any other computing device capable of being configured to carry out the methods described herein.

In some examples, the communication interface may be a network communication interface. In examples in which elements are combined, the communication interface may be a software communication interface, such as those for inter-process communication (IPC). In still other examples, there may be a combination of communication interfaces implemented as hardware, software, and a combination thereof.

Program code may be applied to input data to perform the functions described herein and to generate output information. The output information is applied to one or more output devices, in known fashion.

Each program may be implemented in a high-level procedural, declarative, functional or object-oriented programming and/or scripting language, or both, to communicate with a computer system. However, the programs may be implemented in assembly or machine language, if desired. In any case, the language may be a compiled or interpreted language. Each such computer program may be stored on a storage media or a device (e.g., ROM, magnetic disk, optical disc) readable by a general or special purpose programmable computer, for configuring and operating the computer when the storage media or device is read by the computer to perform the procedures described herein. Examples of the system may also be considered to be implemented as a non-transitory computer-readable storage medium, configured with a computer program, where the storage medium so configured causes a computer to operate in a specific and predefined manner to perform the functions described herein.

Furthermore, the example system, processes and methods are capable of being distributed in a computer program product comprising a computer readable medium that bears computer usable instructions for one or more processors. The medium may be provided in various forms, including one or more diskettes, compact disks, tapes, chips, wireline transmissions, satellite transmissions, internet transmission or downloads, magnetic and electronic storage media, digital and analog signals, and the like. The computer useable instructions may also be in various forms, including compiled and non-compiled code.

Various examples of systems, methods and computer programs products are described herein. Modifications and variations may be made to these examples without departing from the scope of the invention, which is limited only by the appended claims. Also, in the various user interfaces illustrated in the figures, it will be understood that the illustrated user interface text and controls are provided as examples only and are not meant to be limiting. Other suitable user interface elements may be used with alternative implementations of the systems and methods described herein.

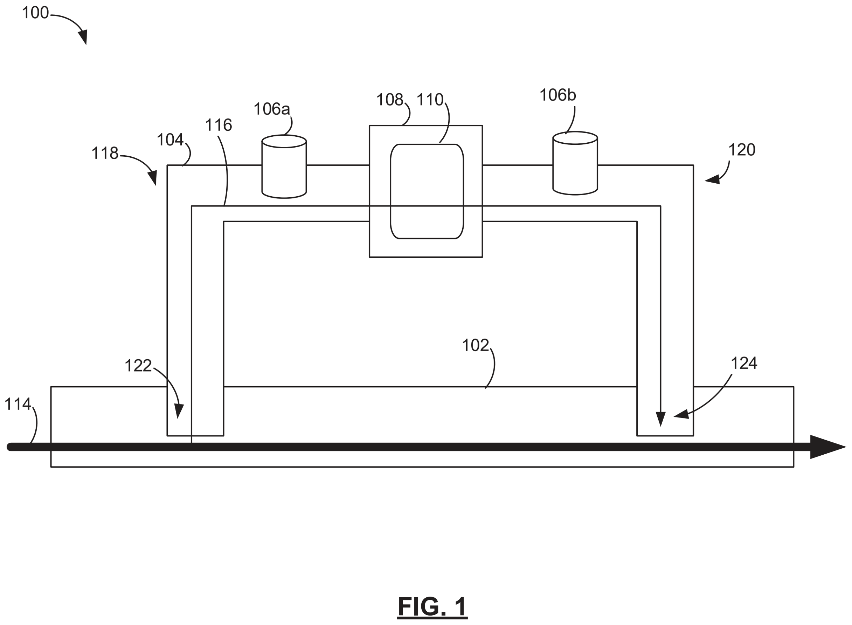

Referring first to FIG. 1, there is shown a side view 100 of an assembly with an extraction member 104 that returns an exhaust gas sample 116 to the exhaust gas flow 114 including two sensors 106 and ammonia capture housing 108 including ammonia capture media 110 in accordance with one or more embodiments.

The extraction member 104 may be an extraction tube, or another hollow member communicating the exhaust gas sample 116 from the exhaust gas flow 114 in the exhaust gas vent 102. The extraction member 104 may be attached to the exhaust vent 102 in a substantially airtight fashion. The extraction member 104 may be generally cylindrical with a first bend 118 and a second bend 120. Alternatively, the extraction member 102 may be constructing from a tube or piping system and assembled with threaded connectors.

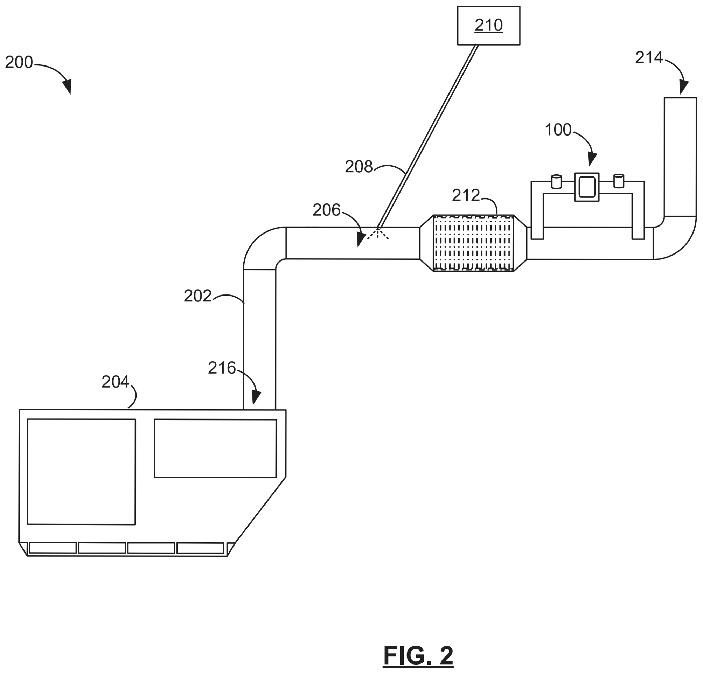

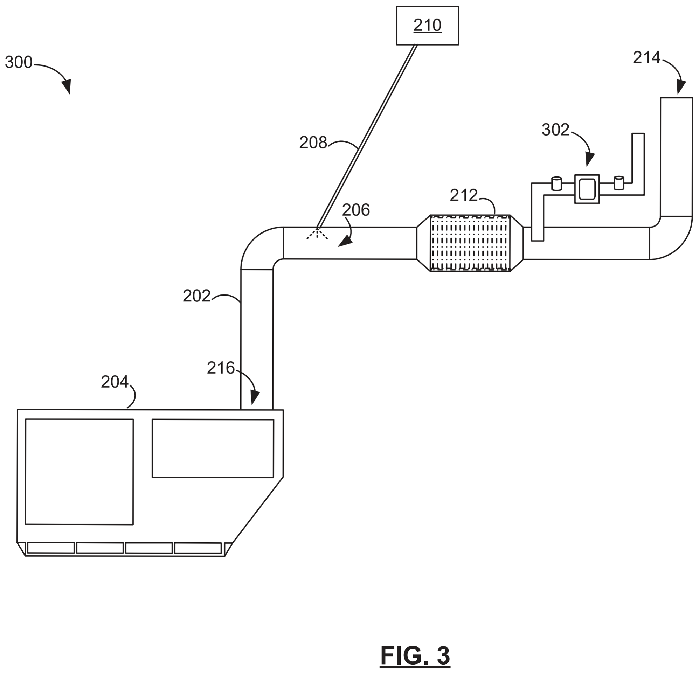

The extraction member 104 includes an inlet end 122 and an outlet end 124. The extraction member 104 includes a pre-capture sensor 106a, an ammonia capture housing 108 including an ammonia capture media 110, and a post-capture sensor 106b. The extraction member 104 may return the exhaust gas sample 116 back into the exhaust gas flow 114 in exhaust vent 102 (see e.g. FIG. 2). Alternatively, the extraction member 104 may exhaust the gas sample 116 to an atmospheric outlet (see e.g. FIG. 3).

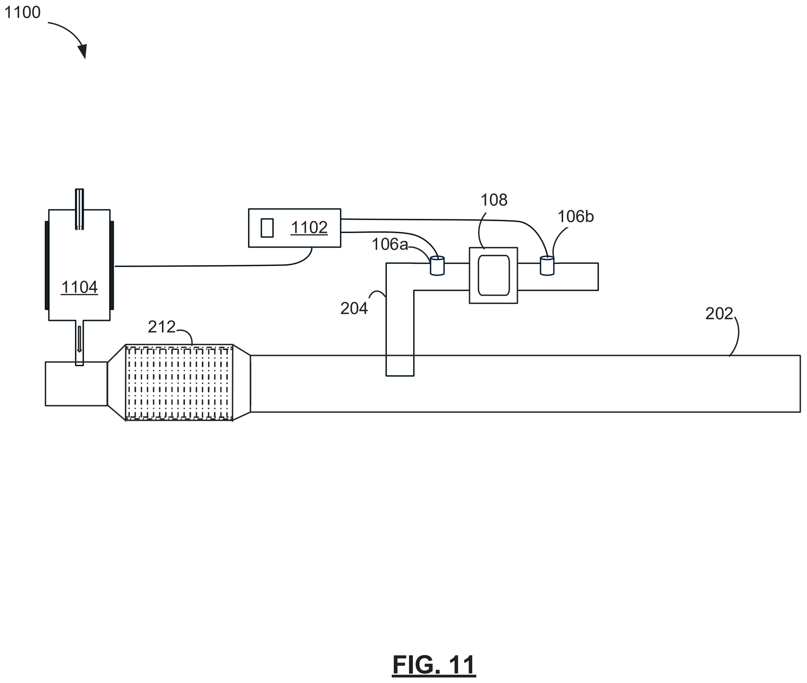

The exhaust gas flow 114 may be in an exhaust vent 102 which communicates the emissions from a combustion device to the environment (see e.g. FIGs. 2-3). The assembly 100 may be upstream or downstream of an SCR device along the exhaust vent 102. The exhaust gas flow 114 may include gaseous ammonia generated by an independent heater (see e.g. FIG. 11) or generated by a liquid urea spray heated within the exhaust vent 102 ahead of the SCR device.

The sensors 106 including pre-capture sensor 106a and post-capture sensor 106b which sense emissions constituents within the exhaust gas sample 116 such as NOₓ emissions. The pre-capture sensor 106a is upstream from the ammonia capture housing 108 and measures at least one upstream NOₓ concentration in the exhaust gas sample. The post-capture sensor 106b is downstream from the ammonia capture housing 108. The sensors 106 may be conventional sensors that measure different constituent NOₓ emissions, for example at least one selected from the group of nitric oxide, nitrogen dioxide, nitric acid, nitrous acid, dinitrogen pentoxide, peroxyacetyl nitrate, alkyl nitrates, peroxyakyl bitrates, nitrate radical, and peroxynitric acid. The sensors 106 may be conventional sensors that have ammonia cross sensitivity, that is, the NOₓ measurements collected are sensitive to the presence of gaseous ammonia. The assembly 100 may further include a heater for heating the pre-capture sensor 106a and the post-capture sensor 106b to an operating temperature. The pre-capture sensor 106a and the post-capture sensor 106b may receive a heating airflow from the inlet end 122 of the extraction member 104. The heating airflow may be the exhaust gas sample 116.

The ammonia capture housing 108 including ammonia capture media 110. In one or more embodiments, the ammonia capture housing 108 may be integrated into the extraction member 104. Alternatively, the ammonia capture housing 108 may be a hollow cylinder, box, or another shape that receives the ammonia capture media 110. The ammonia capture housing 108 may be a sorbent housing, for example, where the ammonia capture media 110 is an ammonia sorbent. The ammonia capture housing 108 may include access means to remove and replace the ammonia capture media 110. The access means may be an substantially airtight door or removable section that provides access for removing and replacing the ammonia capture media 110 when the ammonia capture media 110 is consumed. The ammonia capture media 110 may be phosphoric acid. The ammonia capture media 110 may be a condenser. The ammonia capture housing 108 and the capture media 110 may be heated to an operating temperature by an external heat source. The ammonia capture housing 108 may receive a heating airflow from the inlet end 122 of the extraction member 104. The heating airflow may be the exhaust gas sample 116.

Referring next to FIGs. 2-3, there are shown system views 200 and 300 of the assembly 100 from FIG. 1 with reference to the SCR and an combustion device in accordance with one or more embodiments. The system 200 is an emissions control system for the combustion device 204 having the sensor assembly 100 venting back to the exhaust vent 202. The system 300 is an emissions controls system for the combustion device 204 having the sensor assembly 302 venting to the atmosphere. The exhaust system 200 is in fluid communication with a combustion device 204 in accordance with one or more embodiments. The exhaust system 200 includes an inlet end 216 of the exhaust duct 202 receiving exhaust from the combustion device 204, a selective catalytic reduction (SCR) device 212 in fluid communication with the inlet end 216 of exhaust duct 202, an ammonia generation means 210 supplying liquid urea solution via liquid supply 208 to a spray region 206 where the liquid urea solution is sprayed to generate gaseous ammonia, the sensor assembly 100 (e.g. FIG. 1) and an outlet end 214 of the exhaust duct 202 venting to the atmosphere.

The exhaust from the combustion device 204 progresses through the inlet end 216 of exhaust duct 202, joins the flow of gaseous ammonia from ammonia generation means 210, engages the SCR device 212 to reduce emissions in conjunction with the gaseous ammonia, where an exhaust gas sample is collected by the sensor assembly 100 and then the exhaust gas is vented to the environment at outlet end 214 of exhaust vent 202.

The combustion device 204 may be a boiler, generator or an internal combustion engine. The combustion device 204 may generate an exhaust flow that is received by the inlet end 216 of the exhaust vent 202 for emissions reduction.

In one embodiment, a Diesel Particulate Filter (DPF) may be provided in the exhaust duct either upstream of the SCR device 212 or downstream of the SCR device 212.

The ammonia generation means 210 communicates liquid reductant solution (such as liquid urea solution) via liquid supply 208 into spray region 206. The liquid reductant solution is added into the unpurified exhaust-gas flow with a reductant injector. The reductant injector may be at the spray region 206, or alternatively may be at the SCR device 212 itself. The exhaust gas flow within the exhaust duct heats and converts the reductant agent to a gaseous reductant agent ahead of the SCR device 212. The reductant agent can perform a chemical reaction with the NOₓ in the exhaust gas in order to convert the NOₓ, into N₂ and water vapor in the SCR device 212. The ammonia generation means 210 may include a pump, or a pressurized vessel designed to deliver a flow of liquid reductant such as urea from a reservoir to the injector in either spray region 206 or an injector in SCR device 212. The injector may generate a spray of droplets of the liquid reductant within the spray region 206 of the exhaust vent 202 or the SCR device 212 where it may be converted to a gaseous reductant such as gaseous ammonia by heating. The ammonia supply means 210 may deliver liquid reductant to the gasifier 100 via a valve, or another control mechanism.

The SCR device 212 may be pre-heated using a pre-heat system. The pre-heat system may be the one described in US Provisional Patent Application 63/574,608 which is hereby incorporated by reference in its entirety.

In another embodiment, an ammonia gasifier (see FIG. 11) may be used to generate gaseous that is added into the unpurified exhaust-gas flow ammonia ahead of the SCR device. The ammonia gasifier may be as described in US Provisional Patent application 67/860,868 which is hereby incorporated by reference in its entirety.

The reductant agent may be, for example, aqueous ammonia or a liquid urea solution that is injected and vaporized to form gaseous ammonia.

The exhaust vent 202 receives an exhaust gas flow from the combustion device 204. The exhaust vent 204 may be substantially airtight, and may be a hollow cylinder shape or a squared ducting as known.

The assembly 100 (see e.g. FIG. 1) collects an exhaust gas sample and measures exhaust gas constituents which may be used by a control system to control the operation of the ammonia generation means 210.

Referring system 200, as shown, the assembly 100 is configured to vent the exhaust gas sample back to exhaust vent 202. A one way valve may be positioned at the exhaust end 124 of the assembly in order to ensure that the exhaust gas does not backflow into the assembly 100.

Referring to system 300, as shown, the assembly 302 is configured to vent the exhaust gas sample to the atmosphere.

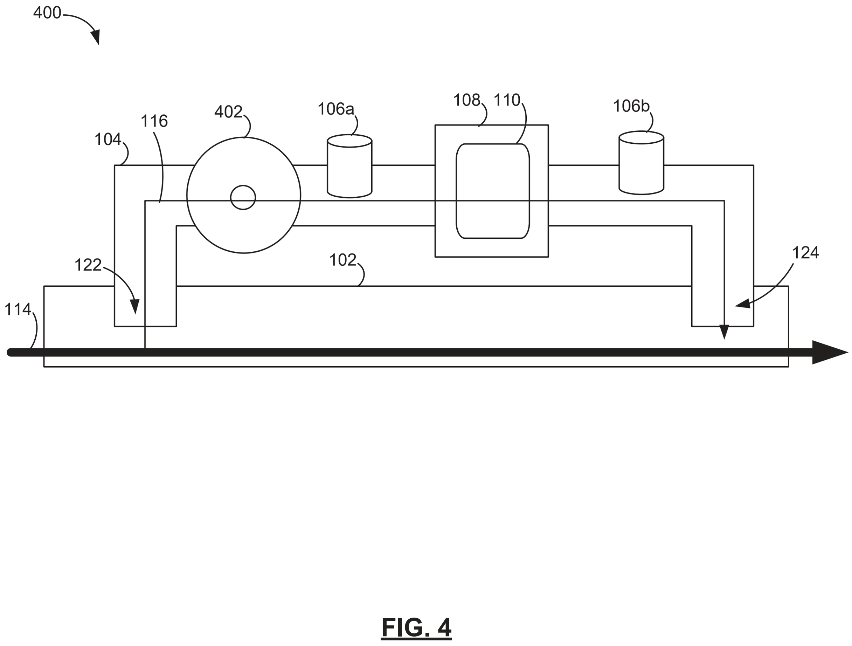

Referring next to FIGs. 4-5, there are shown a side views 400 and 500 of the assembly from FIG. 1 with a pump 402 placed upstream the pre-capture sensor 106a, and a pump 502 place downstream of the post-capture sensor 106b in accordance with one or more embodiments.

The pump 402 may be an electric vacuum pump or fan that may be configured to urge the exhaust sample flow 116 through the inlet 122, the sensors 106, the sorbent 108, and the outlet 124. The pump 402 may be engaged by a control system that can enable and disable it as needed when measurements are collected.

The pump 402 may provide positive pressure upstream the pre-capture sensor 106a. The pump 402 may provide pressure sufficiently higher than the post-capture sensor 106b to overcome the pressure drop across the sorbent 110 so that there is a consistent direction of flow through the sorbent 110.

The pump 502 may be an electric vacuum pump or fan that may be configured to urge the exhaust sample flow 116 through the inlet 122, the sensors 106, the sorbent 108, and the outlet 124. The pump 502 may be engaged by a control system that can enable and disable it as needed when measurements are collected.

The pump 502 may provide positive pressure downstream the post-capture sensor 106b. The pump 502 may provide pressure sufficiently higher than the pre-capture sensor 106a to overcome the pressure drop across the sorbent 110 so that there is a consistent direction of flow through the sorbent 110.

In one embodiment, the functionality of pump 502 may be provided by an eductor 502. An eductor may also be referred to as a jet pump or venturi pump, is a device that uses the flow of a motive fluid (such as air, steam, or liquid) to create a pressure differential and induce the movement of another fluid, in this case inducing exhaust sample flow 116 into the extraction member 104. The principle behind an eductor is the Venturi effect, where a high-velocity jet of motive fluid passes through a constricted section, creating a region of low pressure that draws in and entrains a secondary fluid.

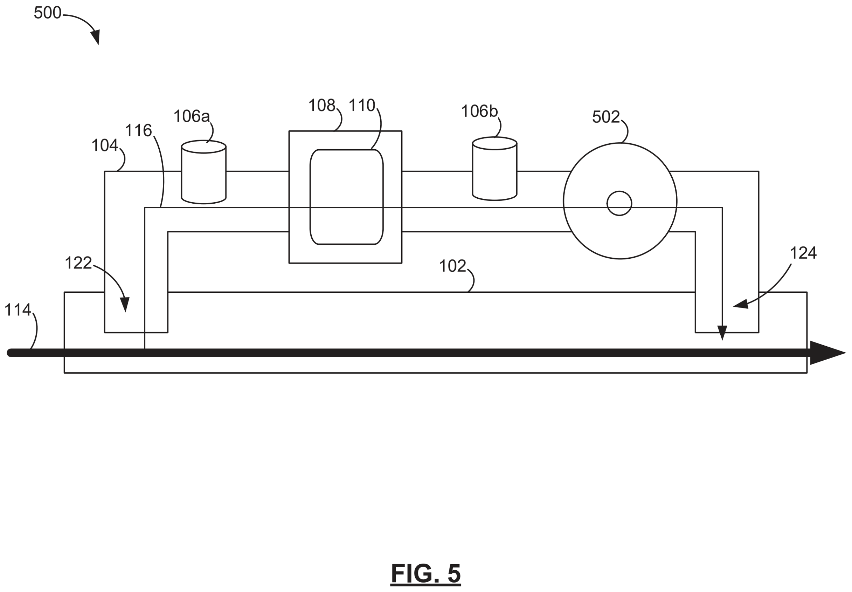

In the context of the exhaust gas extraction system shown in FIG. 5, pump 502 may be used to increase the flow rate of the exhaust gas sample 116 through the extraction member 104, ensuring that the sample passes through the pre-capture sensor 106a, ammonia capture housing 108 (with ammonia capture media 110), and post-capture sensor 106b, before being returned to the exhaust gas flow 114 or vented to the atmosphere.

An eductor could replace pump 502 by utilizing a stream of ambient motive air (or another suitable fluid) to generate low pressure at the outlet end 124 of the extraction member. When the ambient motive air is directed through the eductor, it creates a low-pressure zone that draws the exhaust gas sample 116 through the extraction member 104, across the sensors 106 and ammonia capture media 110, and out through the outlet 124. This method may avoid the need for mechanical moving parts and can be advantageous in high-temperature environments where conventional pumps may be less reliable or require additional cooling.

However, it is important to note that the use of an eductor that introduces ambient motive air into the exhaust stream 114 may dilute the exhaust gas sample 116. This dilution may be accounted for by measuring an oxygen concentration reference (O₂%) in the sample at the pre-capture sensor 106a or the post-capture sensor 106b, allowing for normalization of emission values. The eductor may be sufficiently sized to overcome the pressure drop across the ammonia capture media and maintain a consistent flow rate through the extraction member 104. The measured oxygen concentration reference may further be received by the processor and used to determine ambient air ingress into the extraction member 104.

The exhaust sampling devices in FIGs. 4-5 may exhaust back into the exhaust gas vent 102 as shown, or may exhaust to the ambient atmosphere as shown in FIG. 3.

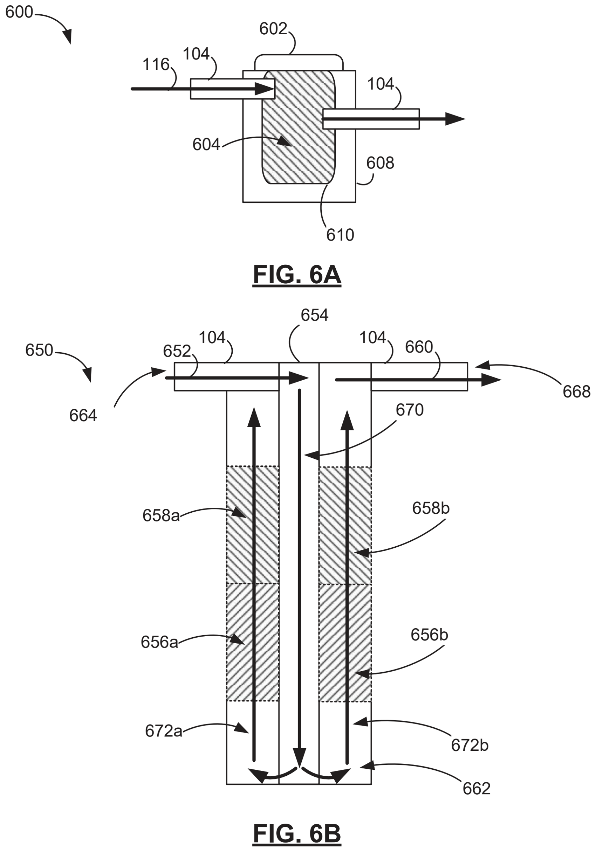

Referring next to FIG. 6A, there is shown a cross-section view 600 of the ammonia capture media including sorbent housing 608 containing the sorbent material 604 in a sorbent chamber 610 in accordance with one or more embodiments.

The sorbent housing 608 may define a closed container and be closed by sorbent housing cap 602. The sorbent housing cap 602 may be secured to sorbent housing 608, for example via threading, latching, or adhesive. The sorbent housing cap 602 may provide for access into the sorbent chamber 610 in order to replace the sorbent material 604 once consumed.

Referring next to FIG. 6B, a cross-section view 650 is shown of an alternate embodiment of the ammonia capture media.

The ammonia capture media 650 includes an inlet 664 and an outlet 668. The inlet 664 receives an incoming exhaust sample flow 652. The outlet 668 provides an outgoing post-capture exhaust sample flow 660 that has a reduced level of gaseous ammonia. The ammonia capture media 650 may be connected as described herein along the exhaust sample vent 104 (see e.g. FIG. 1 and others herein).

The ammonia capture media 650 includes an outer housing 654, an inner channel 670, and one or more outer channels 672. The outer housing 654 may be cylindrical, a rectangular cube, or another shape. The incoming exhaust sample flow 652 enters the inner channel 670 and is directed along the inner channel 670. The end of inner channel 654 has an annular region 662 that opens into the one or more outer channels 672 which return the exhaust sample flow to the outlet 668 as post-capture exhaust sample 660.

The one or more outer channels 672 may each include a flow distribution material 656 and a capture material 658. The flow distribution material 656 may include a plurality of flow distribution beads and the capture material (the sorbent). The flow distribution material 656 may be a wadding material or "burl saddles" which may be mixed with the capture media (sorbent). The flow distribution material 656 increases the working surface area where the exhaust sample flow interacts with the capture material. The supply of capture material (sorbent) may be fed by gravity into the flow distribution material 656 as it is consumed. As discussed herein the flow distribution material 656 and a capture material 658 may be replaceable as it is consumed.

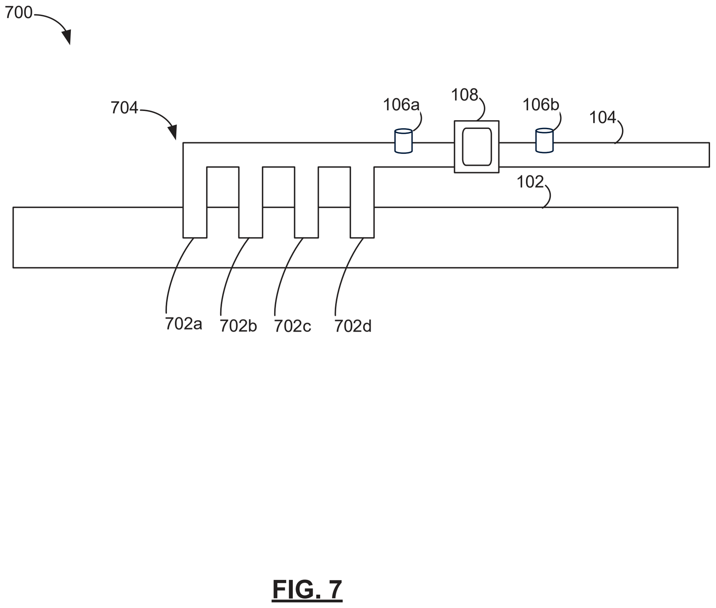

Referring next to FIG. 7, there is shown a side view 700 of an assembly with a plurality of sample inlets in accordance with one or more embodiments.

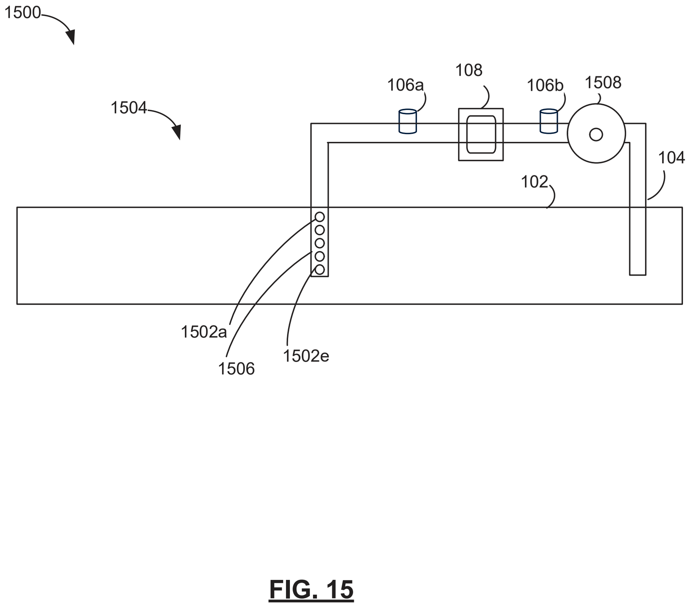

The extraction member 104 may receive the exhaust gas sample from the exhaust gas vent 102 using an exhaust sampling device 704. The exhaust sampling device 704 may collect multiple exhaust sample flows from the exhaust gas vent 102 using a plurality of sampling members 702. While four sampling members are shown 702, it is understood that there may be as few as two sampling members 702 or as many as ten sampling members 702. The plurality of sampling members 702 may each receive an exhaust air sample to provide an improved exhaust profile measurement. These exhaust sampling members 702 may collect exhaust samples from several points along the cross section of the exhaust gas vent 102 and are sampled and averaged by static mixing prior to the gas being introduced to the sensors 106a and 106b. The sampling members 702 may have open ends, or as noted in FIG. 15, may have sampling inlets provided similar to the sampling inlets 1502. The sampling device 704 may have the individual sampling members protrude through the exhaust gas vent 102, or alternatively, may be entirely self contained within the exhaust gas vent 102 and have only a single protrusion through the gas vent 102 for the portion of the sampling device 704 downstream of the sampling members 702 and upstream of the pre-capture sensor 106a.

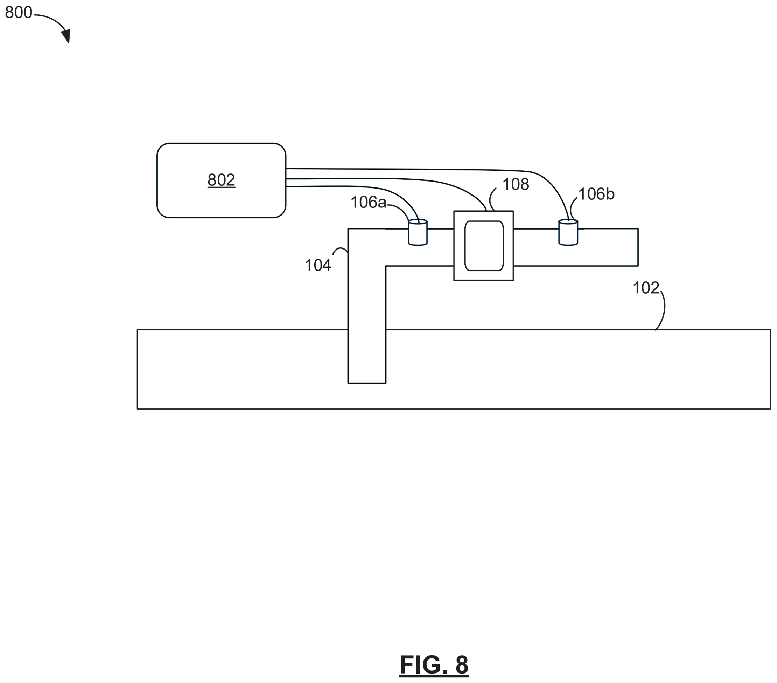

Referring next to FIG. 8, there is shown a side view 800 of the assembly with a heater 802 supplying heat to the pre-capture sensor 106a, post-capture sensor 106b and ammonia capture media 108 in accordance with one or more embodiments.

The heater 802 may be an electric heater. Alternatively, it may be an electric supply that provides electrical power to heating pads proximate to each of the pre-capture sensor 106a, post-capture sensor 106b and ammonia capture media 108. The heater 802 may also include temperature sensors disposed at the pre-capture sensor 106a, post-capture sensor 106b and ammonia capture media 108, and may maintain a temperature set point for each.

The heating of the pre-capture sensor 106a, post-capture sensor 106b and ammonia capture media 108 may reduce the risk of condensation.

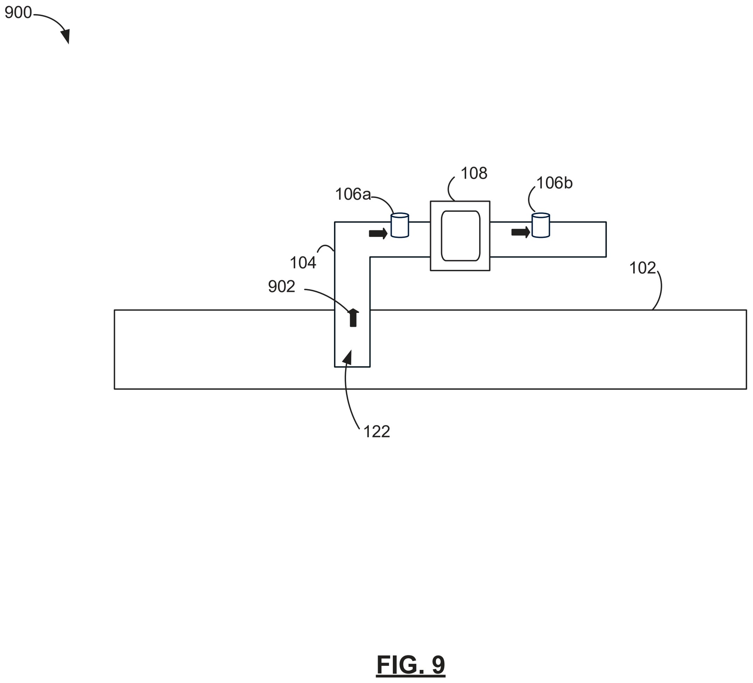

Referring to FIG. 9, there is shown a side view 900 of the assembly with a heating air flow heating the sensors and ammonia capture media in accordance with one or more embodiments.

In one embodiment, the heating of the pre-capture sensor 106a, the post-capture sensor 106b, and the ammonia capture media 108 may be achieved by receiving a heating airflow 902 from the inlet end 122 of the extraction member 104. This may be achieved, for example, with use of a valve or another controlled device permitting exhaust flow to enter the extraction tube 104 for heating. In another embodiment, the exhaust flow sample itself may be used as a heating airflow to heat the pre-capture sensor 106a, the post-capture sensor 106b, and the ammonia capture media 108.

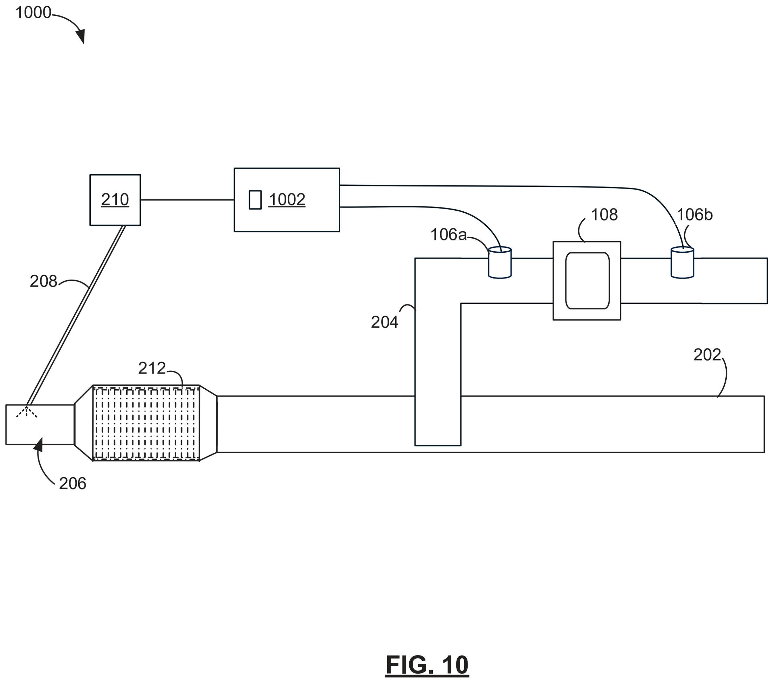

Referring to FIG. 10, there is shown a side view 1000 of the assembly with a processor 1002 in communication with the sensors 106 in accordance with one or more embodiments.

The embodiments herein may provide for an improved method of emission reduction by providing additional and more accurate measurements from sensors 106 to a control system 1002 controlling the emissions reduction process, including an ammonia generation means 210 that delivers liquid urea solution upstream of an SCR device 212.

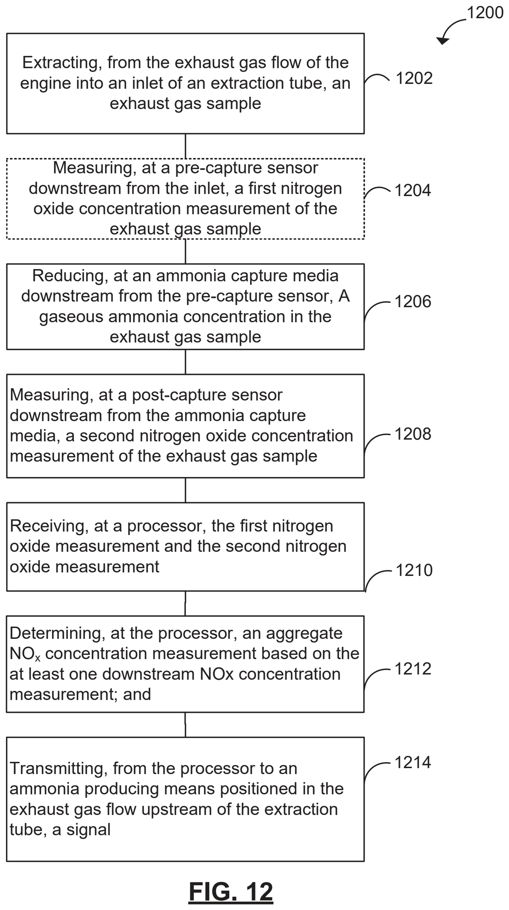

The processor 1002 may implement the control system, and may receive emission constituent measurements from the sensors 106a and 106b as described herein. The processor 1002 may provide the method of FIG. 12. This may include receiving input emission constituent measurements from the sensors 106, and controlling the operation of the ammonia generation means 210 which delivers liquid urea solution into the spray region 206 upstream of SCR device 212.

Referring next to FIG. 11, there is shown a side view 1100 of the assembly of FIG. 10 including an independent ammonia producing means 1104 connected to the processor 1102 in accordance with one or more embodiments.

The independent ammonia producing means 1104 may be an ammonia gasifier as described herein, and may be controlled in terms of temperature and ammonia injection rate by the processor 1102.

The embodiments herein may provide for an improved method of emission reduction by providing additional and more accurate measurements from sensors 106 to a control system 1002 controlling the emissions reduction process, including an independent ammonia generation means 1104 that delivers gaseous ammonia in the exhaust vent 102 upstream of an SCR device 212.

The processor 1102 may implement the control system, and may receive emission constituent measurements from the sensors 106a and 106b as described herein. The processor 1102 may provide the method of FIG. 12. This may include receiving input emission constituent measurements from the sensors 106, and controlling the operation of the independent ammonia generation means 1104 which delivers gaseous ammonia upstream of SCR device 212.

Referring next to FIG. 12, there is shown a method diagram 1200 in accordance with one or more embodiments. The method 1200 is a method of emission reduction in an exhaust gas flow.

At 1202, extracting, from the exhaust gas flow into an inlet of an extraction member, an exhaust gas sample.

Optionally, at 1204, measuring, at an pre-capture sensor positioned between the inlet of the extraction member and the ammonia capture media, at least one upstream NOx concentration measurement of the exhaust gas sample; receiving, at the processor, the at least one upstream NOx measurement; wherein the aggregate NOx concentration measurement may be determined based on the at least one downstream NOx concentration measurement and the at least one upstream NOx concentration measurement.

At 1206, reducing, using an ammonia capture media within the extraction member, gaseous ammonia from the exhaust gas sample.

At 1208, measuring, at a post-capture sensor positioned downstream from the ammonia capture media, at least one downstream NOx concentration measurement of the exhaust gas sample.

At 1210, receiving, at a processor, the at least one downstream NOx measurement.

At 1212, determining, at the processor, an aggregate NOx concentration measurement based on the at least one downstream NOx concentration measurement.

At 1214 transmitting, from the processor to an ammonia producing means positioned in the exhaust gas flow, a control signal based on the aggregate NOx concentration measurement.

Optionally, at least one of the pre-capture sensor and the post-capture sensor may be cross-sensitive to ammonia.

Optionally, the at least one downstream NOx concentration measurement and the at least one upstream NOx concentration measurement may include at least one selected from the group of: nitric oxide and nitrogen dioxide.

Optionally, the at least one downstream NOx concentration measurement and the at least one upstream NOx concentration measurement may further comprise at least one selected from the group of: nitric oxide, nitrogen dioxide, nitric acid, nitrous acid, dinitrogen pentoxide, peroxyacetyl nitrate, alkyl nitrates, peroxyakyl bitrates, nitrate radical, and peroxynitric acid.

Optionally, the method may further include storing in a memory at least one selected from the group of: the aggregate NOx concentration measurement, the at least one upstream NOx concentration measurement and the at least one downstream NOx concentration measurement.

Optionally, the method may further include determining an ammonia concentration based at least one selected from the group of: the aggregate NOx concentration measurement, the at least one upstream NOx concentration measurement and the at least one downstream NOx concentration measurement.

Optionally, the method may further include sending a control signal to a pump receiving the exhaust gas sample.

Optionally, the method may further include determining, at the processor, a state of the ammonia capture media.

Optionally, the method may further include transmitting, from the processor to a network device, the state of the ammonia capture media to a network device.

Optionally, the method may further include determining the control signal for the ammonia producing means based on the aggregate NOx concentration measurement and the state of the ammonia capture media.

Optionally, the method may further include a pre-capture sensor external heat source and a post-capture sensor external heat source; wherein the processor is configured to send a control signal to the first sensor external heat source and post-capture sensor external heat source.

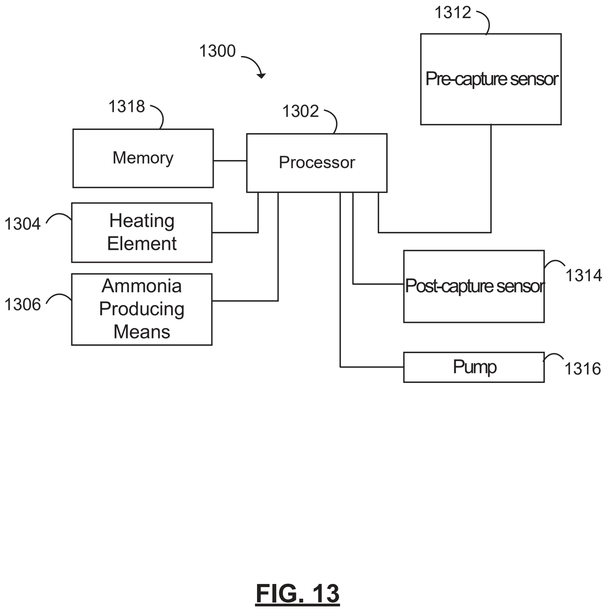

Referring next to FIG. 13, there is shown a device diagram 1300 in accordance with one or more embodiments.

The processor 1302 controls the operation of the emissions system. The processor 1302 can be any suitable processor, controller or digital signal processor that can provide sufficient processing power as is known by those skilled in the art. For example, the processor 1302 may be a PIC, an FPGA, an Arduino, or another general processor. The processor 1302 may be an Intel^{®} processor, an ARM^{®} processor or a microcontroller.

The memory 1318 may store software code or programs that processor loads in order to provide the control system described herein, such as a program that implements the method of FIG. 12. The memory 1318 can include RAM, ROM, one or more hard drives, one or more flash drives or some other suitable data storage elements such as disk drives, etc. The memory 1318 may be used to store an operating system and programs as is commonly known by those skilled in the art.

The processor 1302 may be connected to and may control one or more heating elements 1304 (see e.g. FIG. 8) to control the heating of the sensors and the ammonia capture means 1306.

The processor 1302 may be connected to and may control the ammonia producing means, for example a pump 1316 that delivers liquid urea solution (see e.g. FIG. 10) or alternatively an independent heating ammonia generation means (see e.g. FIG. 11).

The processor 1302 may be connected to and may control the operation of a pump 1316, for example, for urging the exhaust sample airflow through the extraction member (see e.g. FIGs. 4-5).

The processor 1302 may receive emission constituent measurements from a pre-capture sensor 1312 as described herein.

The processor 1302 may receive emission constituent measurements from a post-capture sensor 1314 as described herein.

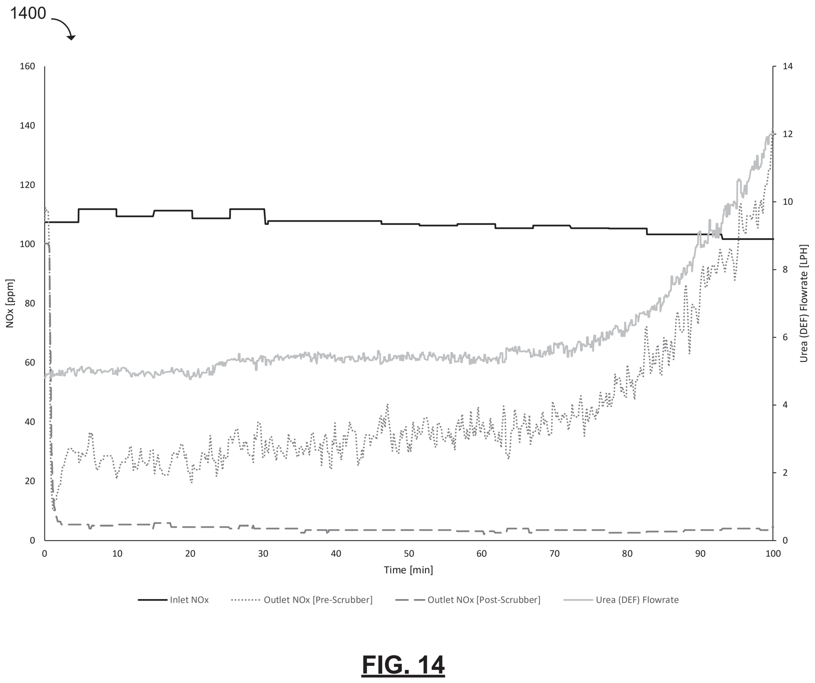

Referring next to FIG. 14, there is shown a results diagram 1400 showing the performance of the sensor assembly including the ammonia capture media.

The results 1400 showcase the measurement stability achieved on the NOx sensor downstream from the ammonia capture media. Comparing the "Outlet NOx [Post-Scrubber]" with the "Outlet NOx [Pre-Scrubber]" a significant stabilization effect achieved is shown for the measurements made by the sensor downstream from the sorbent, with gaseous ammonia removed.

Referring next to FIG. 15 there is shown a side view of another assembly in accordance with one or more embodiments.

The extraction member 104 may receive the exhaust gas sample from the exhaust gas vent 102 using an exhaust sampling device 1504. The exhaust sampling device 1504 may be provided as an extraction tube or hollow member 1506 having multiple sampling inlets 1502 collecting exhaust gas. The hollow member 1506 may have one or more sampling inlets 1502 and may be sealed at the end positioned within exhaust gas vent 102. While four sampling inlets 1502 are shown, it is understood that there may be as few as two sampling inlets 1502 or as many as ten sampling inlets 1502. The plurality of sampling inlets 1502 may each receive an exhaust air sample to provide an improved exhaust profile measurement. These exhaust sampling inlets 1502 may collect exhaust samples from several points along the cross section of the exhaust gas vent 102 and are sampled and averaged by static mixing prior to the gas being introduced to the sensors 106a and 106b. The plurality of sampling inlets 1502 in member 1506 may be provided in parallel across the diameter of exhaust gas vent 102. The plurality of sampling inlets 1502 may be positioned upstream from the hollow member 1506, at the same position as the member 1506 (as shown in FIG. 15), or downstream from the member 1506. The embodiment in FIG. 7 may be combined with the embodiment in FIG. 15 where exhaust inlets are provided on the sampling members 702 of a sampling assembly similar to 704, where the sampling members have closed ends.

The exhaust sampling device 1504 may have a pump or eductor 1508 provided to urge the exhaust sample through the extraction member 104, for example as described in further detail in FIG. 5.

The exhaust sampling device 1504 may exhaust back into the exhaust gas vent 102 as shown, or may exhaust to the ambient atmosphere as shown in FIG. 3.

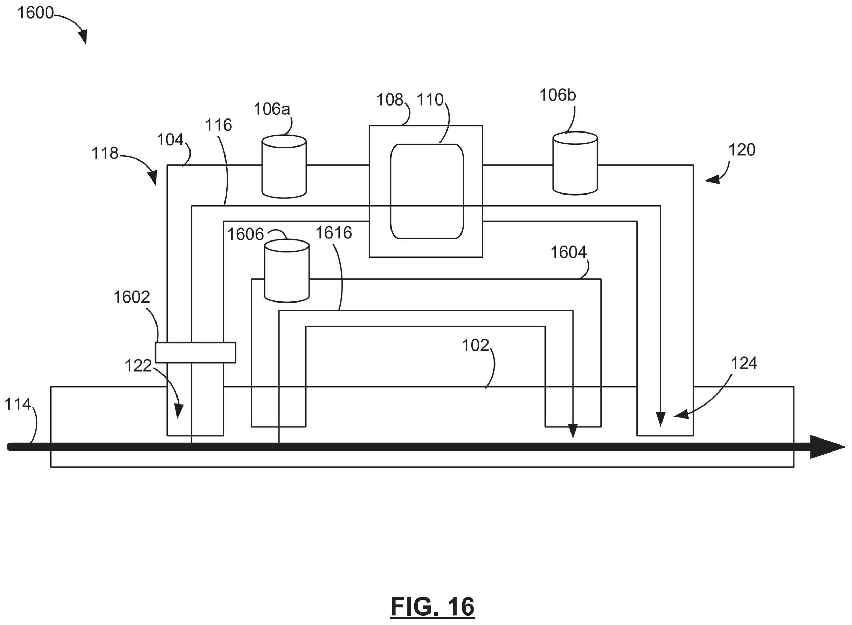

Referring next to FIG. 16 there is shown another side view 1600 of an assembly with the extraction member that returns the exhaust gas sample to the exhaust gas flow including two sensors and ammonia capture media, and additionally an independent extraction member 1604 in accordance with one or more embodiments. In this embodiment, the use of the ammonia capture media may be extended by measuring periodically, or based on the sending of a sensor 1606 without the benefit of an ammonia capture media.

In one embodiment, an additional independent extraction member 1604. In this embodiment, a valve or isolation device 1602 may be used to open the extraction member 104 to the exhaust gas flow 114 in exhaust vent 102. This valve or isolation device 1602 may be provided to isolate the two sensors 106 and the ammonia capture media 110 to improve the lifespan of the ammonia capture media 110.

The independent extraction member 1604 is provided with sensor 1606 and receives independent sample airflow 1616 received from exhaust flow 114. The sensor 1606 may transmit a signal to a processor controlling the valve 1602. The sensor 1606 may detect the increase in emissions (either due to ammonia cross-sensitivity of the sensor 1606 or from NOx emissions within the sample flow 1616) from the exhaust gas flow 114 relatively, and the processor, based on the receipt of the sensor signal from the sensor 1606 may open the valve 1602 in order to determine a more precise value for the emissions in the exhaust gas flow 114. This independent extraction member 1604 may thus provide for programmatic control of the sampling device including the ammonia capture media 110. This may improve the lifespan of the ammonia capture media 110. This may provide for independent control on an as-needed basis. Where the extraction member 104 includes an eductor or pump, the processor may similarly engage the eductor or pump to perform measurements using sensors 106a and 106b based on a signal from the potentially cross-sensitive sensor 1606.

In an alternate embodiment, the valve 1602 may be engaged according to a timer or a regular schedule, for example, 1 minute per hour or every 10 minutes.

The opening and closing of the valve 1602 and the engagement of the eductor or pump may be based on a detection by the processor of an adverse condition such as a slip - i.e. when too much urea solution has been injected into the exhaust vent 102 at the SCR device. It is noted that other conditions may also cause slip - for example, urea moving by the SCR device and not being catalyzed.

If more and more urea is injected and emissions increase, such a situation is a negative indicator about the operation of the overall emission reduction device. In this case, the extraction member 104 may be engaged by opening valve 1602 to sample using the post-capture sensor 106b.

The present invention has been described here by way of example only. Various modification and variations may be made to these exemplary embodiments without departing from the spirit and scope of the invention, which is limited only by the appended claims.

All publications, patents and patent applications are herein incorporated by reference in their entirety to the same extent as if each individual publication, patent or patent application was specifically and individually indicated to be incorporated by reference in its entirety.

## Claims

1. A sensor apparatus for measuring emissions, the apparatus comprising:
- an extraction member in fluid communication with the exhaust gas flow, the extraction member having an inlet end and an outlet end, the inlet end receiving an exhaust gas sample from the exhaust gas flow;
- an ammonia capture media positioned downstream of the inlet end within the extraction member, the ammonia capture media comprising a sorbent configured to remove ammonia from the exhaust gas sample;
- a post-capture sensor positioned downstream of the ammonia capture media, the post-capture sensor configured to measure at least one downstream NOₓ concentration measurement in the exhaust gas sample of the exhaust gas flow; and
- wherein the post-capture sensor are cross-sensitive to ammonia.

2. The apparatus of claim 1 further comprising:
- an pre-capture sensor positioned between the inlet end and the ammonia capture media and configured to measure at least one upstream NOₓ concentration measurement in the exhaust gas sample; and
- a collector assembly connected to the inlet end, the collector assembly comprising at least two sample inlets in communication with the exhaust gas flow, a first sample inlet in the at least two sample inlets receiving a first portion of the exhaust gas sample at a first location of the exhaust gas flow and a second sample inlet in the at least two sample inlets receiving a second portion of the exhaust gas sample at a second location of the exhaust gas flow.

3. The apparatus of any one of claims 1 to 2, further comprising an eductor or a pump configured to increase a flow rate of the exhaust gas sample.

4. The apparatus of any one of claims 1 to 3, wherein the at least one downstream NOx concentration measurement and the at least one upstream NOₓ concentration measurement comprise at least one selected from the group of: nitric oxide and nitrogen dioxide, the at least one downstream NOₓ concentration measurement and the at least one upstream NOₓ concentration measurement further comprise at least one selected from the group of: nitric acid, nitrous acid, dinitrogen pentoxide, peroxyacetyl nitrate, alkyl nitrates, peroxyakyl bitrates, nitrate radical, and peroxynitric acid.

5. The apparatus of any one of claims 1 to 4, wherein the ammonia capture media further comprises:
- a removable sorbent housing in communication with the exhaust gas;
- wherein the sorbent is positioned within the removable sorbent housing .

6. The apparatus of any one of claims 1 to 5, wherein the sorbent is one of phosphoric acid or a condenser.

7. The apparatus of claim 1, wherein the pre-capture sensor, the post-capture sensor, and the ammonia capture housing are heated to an operating temperature by an external heat source, optionally the exhaust gas sample.

8. A method for reducing emissions in an exhaust gas flow, the method comprising:
- extracting, from the exhaust gas flow into an inlet of an extraction member, a first exhaust gas sample;
- reducing, using an ammonia capture media within the extraction member, a gaseous ammonia concentration in the first exhaust gas sample;
- measuring, at a post-capture sensor positioned downstream from the ammonia capture media, at least one downstream NOₓ concentration measurement of the first exhaust gas sample;
- receiving, at a processor, the at least one downstream NOₓ measurement;
- determining, at the processor, an aggregate NOₓ concentration measurement based on the at least one downstream NOx concentration measurement;
- transmitting, from the processor to an ammonia producing means positioned in the exhaust gas flow, a control signal based on the aggregate NOx concentration measurement; and
- wherein the post-capture sensor are cross-sensitive to ammonia.

9. The method of claim 8 further comprising:
- measuring, at an pre-capture sensor positioned between the inlet of the extraction member and the ammonia capture media, at least one upstream NOₓ concentration measurement of the first exhaust gas sample;
- receiving, at the processor, the at least one upstream NOₓ measurement;
- wherein the aggregate NOₓ concentration measurement is determined based on the at least one downstream NOₓ concentration measurement and the at least one upstream NOₓ concentration measurement.

10. The method of claim 9, wherein the at least one downstream NOₓ concentration measurement and the at least one upstream NOₓ concentration measurement comprise at least one selected from the group of: nitric oxide and nitrogen dioxide, the at least one downstream NOₓ concentration measurement and the at least one upstream NOₓ concentration measurement comprise at least one selected from the group of: nitric oxide, nitrogen dioxide, nitric acid, nitrous acid, dinitrogen pentoxide, peroxyacetyl nitrate, alkyl nitrates, peroxyakyl bitrates, nitrate radical, and peroxynitric acid.

11. The method of claim 10, further comprising:
- storing in a memory at least one selected from the group of: the aggregate NOₓ concentration measurement, the at least one upstream NOₓ concentration measurement and the at least one downstream NOₓ concentration measurement;
- determining an ammonia concentration based at least one selected from the group of: the aggregate NOₓ concentration measurement, the at least one upstream NOₓ concentration measurement and the at least one downstream NOₓ concentration measurement; and
- sending a control signal to a pump receiving the first exhaust gas sample.

12. The method of claim 11, further comprising:
- determining, at the processor, a state of the ammonia capture media;
- transmitting, from the processor to a network device, the state of the ammonia capture media to a network device; and
- determining the control signal for the ammonia producing means based on the aggregate NOx concentration measurement and the state of the ammonia capture media.

13. The method of claim 12, further comprising:
- a pre-capture sensor external heat source and a post-capture sensor external heat source;
- wherein the processor is configured to send a control signal to the first sensor external heat source and post-capture sensor external heat source.

14. The method of claim 8, further comprising:
- receiving a signal from a secondary sensor positioned in a second exhaust gas sample;
responsive to the signal from the secondary sensor, sending a signal to an isolation device upstream from the ammonia capture media, the signal to the isolation device permitting the first exhaust gas sample to flow.

15. A system for reducing emissions in an exhaust gas flow, the system comprising:
- an ammonia capture media in an extraction member, an inlet end of the extraction member in fluid communication with the exhaust gas flow and receiving a first exhaust gas sample from the exhaust gas flow, the ammonia capture media for reducing gaseous ammonia in the first exhaust gas sample;
- a post-capture sensor positioned downstream of the ammonia capture media, the post-capture sensor configured to measure at least one downstream NOₓ concentration measurement of the first exhaust gas sample;
- a processor in communication with the post-capture sensor and an ammonia generation means the processor configured to perform the method of any one of claims 8 to 14;
- wherein at least one of the pre-capture sensor and the post-capture sensor are cross-sensitive to ammonia.

16. The system of claim 15 further comprising:
- an isolation device upstream from the ammonia capture media controlling the first exhaust gas sample;
- a secondary sensor positioned in a second exhaust gas sample; and
- wherein the processor is further configured to:
- receive a signal from the secondary sensor; and
- responsive to the signal from the secondary sensor, send a signal to the isolation device to permit the first exhaust gas sample to flow.

17. The system of claim 15 further comprising:
- a secondary sensor positioned in a second exhaust gas sample; and
- wherein the processor is further configured to:
- receive a signal from the secondary sensor; and
- responsive to the signal from the secondary sensor, send a signal to the pump or eductor to permit the first exhaust gas sample to flow.
